# EUROPEAN PATENT SPECIFICATION

(11) **EP 3 164 135 B1**
(45) Date of publication and mention of the grant of the patent: **23.10.2019**
(21) Application number: 15762707.6
(22) Date of filing: 02.07.2015
(51) Int. Cl.: A61K 31/575, A61P 31/12

(54) **OXYSTEROLS FOR USE IN THE TREATMENT AND PREVENTION OF DISEASES CAUSED BY VIRUSES**
OXYSTEROLE ZUR VERWENDUNG BEI DER BEHANDLUNG UND PRÄVENTION VON VIRUSKRANKHEITEN
OXYSTÉROLS DESTINÉS AU TRAITEMENT ET À LA PRÉVENTION DE MALADIES PROVOQUÉES PAR DES VIRUS

(30) Priority: 02.07.2014 IT TO20140533
(43) Date of publication of application: 10.05.2017
(73) Proprietor: Lembo, David, 10136 Torino (IT); Poli, Giuseppe Leone Graziano Enrico, 10083 Favria (IT); Civra, Andrea, 10042 Nichelino (IT)
(72) Inventor: LEMBO David, Torino 10136 (IT); POLI Giuseppe Leone Graziano Enrico, 10124 Turin (IT); CIVRA Andrea, 10042 Nichelino (TO) (IT); CAGNO Valeria, 1203 Geneve (CH)
(74) Representative: Casciano, Lidia Giulia Rita
(86) International application number: PCT/IB2015/055000
(87) International publication number: WO 2016/001870

(56) References cited:
- EP-A1- 2 522 350
- WO-A1-2013/166503
- MATHIEU BLANC ET AL: "The Transcription Factor STAT-1 Couples Macrophage Synthesis of 25-Hydroxycholesterol to the Interferon Antiviral Response", IMMUNITY, vol. 38, no. 1, 1 January 2013 (2013-01-01), pages 106-118, XP055154057, ISSN: 1074-7613, DOI: 10.1016/j.immuni.2012.11.004
- IWAMOTO MASASHI ET AL: "Evaluation and identification of hepatitis B virus entry inhibitors using HepG2 cells overexpressing a membrane transporter NTCP", BIOCHEMICAL AND BIOPHYSICAL RESEARCH COMMUNICATIONS, ACADEMIC PRESS INC. ORLANDO, FL, US, vol. 443, no. 3, 14 December 2013 (2013-12-14), pages 808-813, XP028815147, ISSN: 0006-291X, DOI: 10.1016/J.BBRC.2013.12.052
- MOOG C ET AL: "Oxysterols, but not cholesterol, inhibit human immunodeficiency virus replication in vitro", ANTIVIRAL CHEMISTRY & CHEMOTHERAPY, INTERNATIONAL MEDICAL PRESS, vol. 9, no. 6, 1 January 1998 (1998-01-01) , pages 491-496, XP009181327, ISSN: 0956-3202
- ANDREA CIVRA ET AL: "Inhibition of pathogenic non-enveloped viruses by 25-hydroxycholesterol and 27-hydroxycholesterol", SCIENTIFIC REPORTS, vol. 4, 15 December 2014 (2014-12-15), page 7487, XP055227043, DOI: 10.1038/srep07487

## Description

### TECHNICAL FIELD

The present invention relates to oxysterols for use in the treatment and prevention of infections caused by viruses, in particular naked viruses.

### BACKGROUND ART

Viruses are obligate intracellular parasites and represent an important category of pathogenic infectious agents for humans. According to the structure of the virion, viruses are grouped into two categories: enveloped viruses and naked viruses. The former are characterised by a lipoproteic membrane (viral envelope) which covers a proteic shell (capsid or nucleocapsid) containing the viral genoma (consisting of nucleic acid, DNA or RNA); the latter consist solely of capsid and nucleic acid (DNA or RNA). In order to generate a progeny, viruses must necessarily parasite a host cell, depending on it for their macromolecular synthesis and for the production of energy.

The naked viruses include some important etiological agents of infectious diseases widespread in humans and in animals such as rotaviruses, rhinoviruses and papillomaviruses.

Human rotaviruses are the most widespread etiological agents of viral gastroenteritis in infants and children. The acute diarrhoea caused by rotaviruses represents a global health problem: rotaviruses cause 114 million cases of diarrhoea, which result in 24 million medical consultations and 2.4 million hospital admissions every year (1). Worldwide, rotavirus infections lead to approximately 500,000 deaths; 5% of deaths in children under the age of 5 can be attributed to diarrhoea-related diseases(2). Currently, two preventive vaccines are available on the market (3,4): Rotarix (GlaxoSmithKline) and RotaTeq (Merck). The introduction of RotaTeq in 2006 and Rotarix in 2008 in the United States infant vaccination programme has led to a substantial reduction in terms of hospitalization associated with rotavirus. The results of the clinical trials indicate an 85% effectiveness of Rotarix and RotaTeq vis-à-vis serious gastroenteritis. On the other hand, the clinical trials indicate that the vaccines are much less effective in developing countries, for reasons which are not yet fully clear. Today, specific antiviral drugs are not available on the market, mainly due to a lack of understanding of the molecular mechanisms underlying the diarrhoea induced by the virus. These considerations point to the urgent need to develop specific inhibitors, able to prevent and/or treat gastroenteritis in both humans and domestic animals such as cows, horses and pigs.

Human rhinoviruses are viruses with a single strand positive sense RNA, in the family Picornaviridae. They are responsible for at least 50% of cases of common cold (5,6). The common cold is the most frequent human disease and affects all populations worldwide. Although the disorders associated with this disease are not serious, it has a high economic and social impact (5,6,8). Furthermore, rhinovirus infections have often been associated with the exacerbation of more serious diseases of the lower respiratory tract, such as asthma and chronic obstructive pulmonary disease (COPD) (9,10). A prevention strategy based on the production and administration of a vaccine is not feasible because more than 150 different rhinovirus serotypes exist and the antibody cross reactivity induced by them is low (11). Therefore, above all in order to reduce the economic and social impact and the incidence of rhinovirus infections in patients with asthma or COPD, the development of agents active against the rhinovirus is an urgent need (12) .

The papillomaviruses are naked DNA viruses, able to infect mainly cells of the polystratified squamous epithelium causing proliferative lesions in both humans and animals. In humans, the HPV subtypes defined at low risk (HPV subtype 6 and 11 in 90% of cases) are responsible for a wide range of benign proliferative lesions (13), in the anogenital and oropharyngeal districts (condylomas and papillomas). However, infection by a group of human papillomaviruses - defined at high risk - is associated with ano-genital and head-neck cancer. Of these, cervical cancer is a malignant tumour with a high incidence worldwide. In fact, half a million new cases and approximately 250,000 deaths are recorded every year (14). The majority of these cases are associated with one or more oncogenic HPV subtypes including HPV 16, 18, 31, 33 and 45 (15). The high-risk HPVs can also cause tumours of the vulva, vagina, penis, anus and perianal region, and approximately 20% of head and neck tumours (16). Currently, two vaccines are available for the prevention of high-risk HPV16 and 18 infections. A certain cross reactivity of the vaccines against other subtypes has been observed, but it is not sufficient to provide full cross protection against all the oncogenic subtypes (17). However, the high cost of the vaccines and the logistical problems involved in their distribution and administration represent important limiting factors for the vaccination campaigns in developing countries in which cervical cancer has a high incidence. Furthermore, no data are currently available concerning the long-term effectiveness of this prophylaxis "active in the vaccinated population (17-19), since this vaccine has been introduced only recently and is administered before the person becomes sexually active. For these reasons," the wide range of women to whom the preventive vaccine has not been administered remain exposed and vulnerable to HPV. Overall, this means that also in the era of vaccination against HPV, the identification of effective inhibitors of HPV infection is highly desirable, for use in particular in women with abnormal or positive smear test results or in whom an oncogenic HPV infection is identified.

EP2522350 describes the use of 7beta-hydroxycholesterol for the treatment of hepatitis A.

Moog et al. describe the use of 7beta-hydroxycholesterol, 25-hydroxycholesterol and 7,25-dihydroxycholesterol for the treatment of HIV infections.

WO2013166503 describes the use of 25-hydroxycholesterol for the treatment of infections caused by enveloped viruses. However, this publication illustrates that the 25-hydroxycholesterol is not effective on naked viruses, for example the adenovirus 5 (Liu SY et al. Immunity 38, 92-105, 2013) .

The use of hydroxysterols for the treatment of viral infections is further disclosed by Mathieu Blanc et al. (Immunity, vol. 38, no. 1 (2013-01-01)) and Iwamoto Masashi et al. (Bioch. Bioph. Research Comm., vol. 443, no. 3, (2013-12-14)).

### DISCLOSURE OF INVENTION

The object of the present invention is therefore to provide antiviral compounds which can be applied in the prevention and treatment of infections caused by naked viruses such as rotavirus, rhinovirus and papillomavirus.

Said object is achieved by the present invention, relative to compounds according to claim 1.

The oxysterols, subject of the present invention, belong to a family of molecules with 27 carbon atoms originating from the enzymatic and non-enzymatic oxidisation of cholesterol which, with respect to cholesterol, contain an additional group, namely a hydroxyl or epoxy or ketone group on the nucleus of the sterol and/or a hydroxyl group on the side chain (20,21).

For many years, oxysterols were considered mainly in terms of their physiological role, for example, in the synthesis of bile acids, in the biosynthesis of steroid hormones, in transport of the sterols and more generally in genic regulation. More recently, the potential contribution of particular oxysterols in the pathogenesis and progression of major chronic human diseases associated with inflammation has been studied (22).

According to a first aspect of the invention, oxysterols of formula (I) are provided: 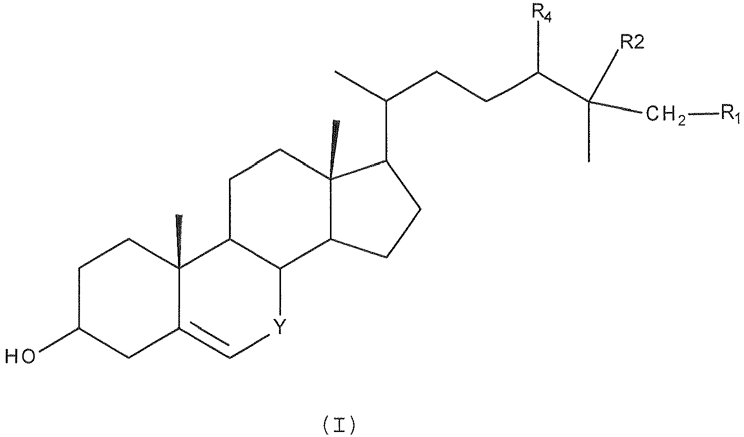 wherein R1 is chosen from the group consisting of H and OH, R2 is chosen from the group consisting of H and OH, R4 is chosen from the group consisting of H, OH, Y is chosen from the group consisting of CHR3 and C=O, and R3 is chosen from the group consisting of H and OH; they are effective in the treatment or prevention of infections caused by a naked virus, in particular selected from the group consisting of rotavirus, rhinovirus and papillomavirus. More preferably the infection is selected from the group consisting of gastroenteritis, rhinopharyngitis (cold), asthma exacerbation, chronic obstructive pulmonary disease exacerbation, genital condyloma, cervical cancer, vulvar cancer, vaginal cancer, penile cancer, anal cancer, perianal cancer, head and neck cancer, warts, papillomas, recurrent respiratory papillomatosis.

In particular, in one embodiment of the present invention, the oxysterol is chosen from the group consisting of 24-hydroxycholesterol, 25-hydroxycholesterol, 27-hydroxycholesterol, 7alpha-hydroxycholesterol, 7beta- hydroxycholesterol, 7,25-di hydroxycholesterol and 7-ketocholesterol.

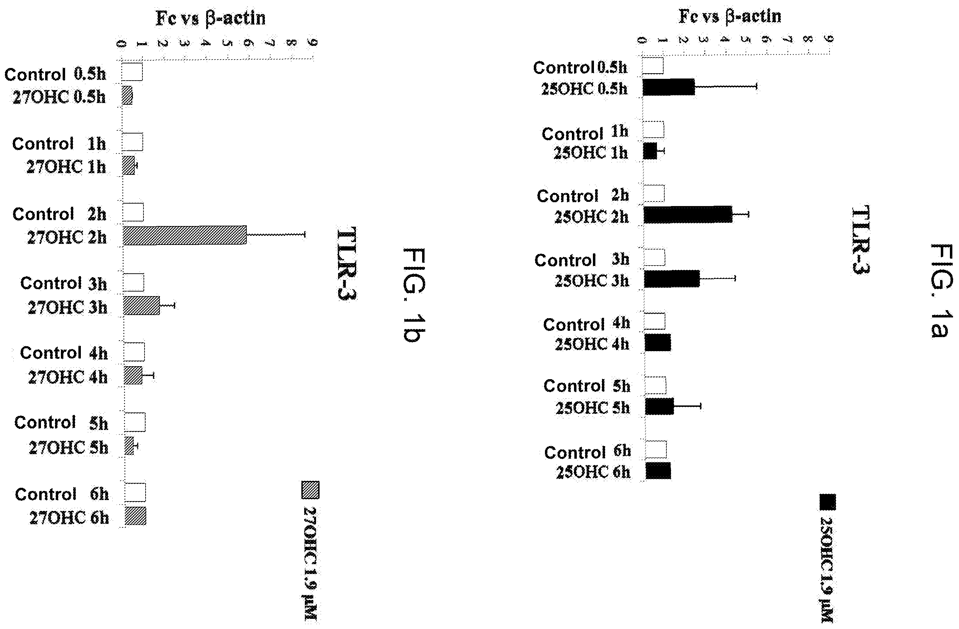

Surprisingly it has been found that certain oxysterols are able to activate the innate immune response and induce the adaptive immune response to the infection of cells and tissues by viral agents, determining the hyperexpression of some components of the family of receptors defined as Toll Like Receptors (TLRs), molecules with a protagonist role in orchestrating the antivirus immune response of the organism (23). Namely, 27-hydroxycholesterol (27OHC) and 25-hydroxycholesterol (25OHC) induce in CaCo2 intestinal cells a marked hyperexpression of TLR3, the receptor for RNA viruses (figures 1a-1b). With reference to the DNA viruses, in particular the papilloma virus, 27-hydroxycholesterol (27OHC) and 7β-hydroxycholesterol (7βOHC) have proved to be active in inducing TLR4, a receptor which they specifically activate, in SH-SY5Y neuroblastoma cells (24) and 27OHC in macrophage cell lines.

The stimulation of the TLRs generates a cascade of molecular events that terminate with activation of the nuclear factor-kB (NF-kB) transcription factor and various interferons (IFNs), with consequent amplification of the inflammatory response of the organism (25). It is worth noting that all the above oxysterols are well-known activators of NFkB in various cell types (26).

Among the oxysterols of the invention with proven viral activity, 25OHC and 27OHC are optimal ligands of LXR (nuclear receptors of primary importance in physiology, since they activate various cell signalling pathways), while other oxysterols such as 7-ketocholesterol have no ligand activity; this demonstrates that the antiviral effect of the oxysterols of the invention is not necessarily or, at least, not entirely dependent on the involvement of said nuclear receptors.

Therefore, on the basis of the experimental results obtained and reported here, it is reasonable to assume that the antiviral effect of the oxysterols of the invention is not a mere surface effect, i.e. limited to the plasma membrane of the target cell, but depends also on the capacity of these compounds to activate multiple cell signalling pathways. In a second aspect of the invention, a pharmaceutical composition is furthermore provided comprising an oxysterol of formula (I): 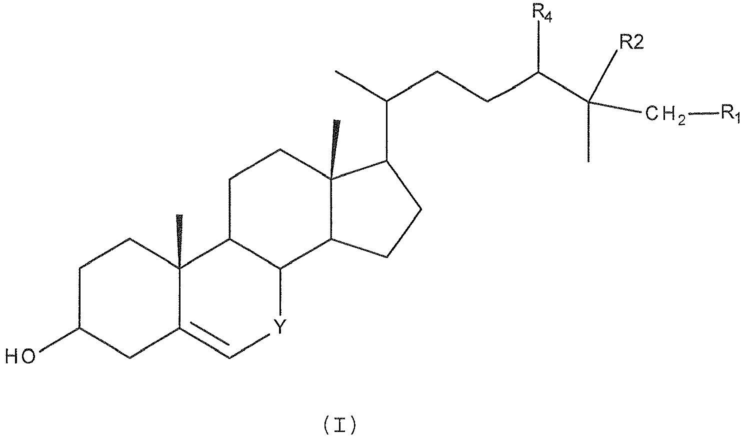 wherein R1 is chosen from the group consisting of H and OH, R2 is chosen from the group consisting of H and OH, R4 is chosen from the group consisting of H, OH, Y is chosen from the group consisting of CHR3 and C=O, and R3 is chosen from the group consisting of H and OH, and at least one pharmaceutically acceptable excipient for use in the treatment and prevention of infections caused by a naked virus, in particular selected from the group consisting of rotavirus, papillomavirus and rhinovirus. In particular the infection is selected from the group consisting of gastroenteritis, rhinopharyngitis (cold), asthma exacerbation, chronic obstructive pulmonary disease exacerbation, genital condyloma, cervical cancer, vulvar cancer, vaginal cancer, penile cancer, anal cancer, perianal cancer, head cancer, neck cancer, warts, papillomas, recurrent respiratory papillomatosis.

In particular, in one embodiment of the present invention, the oxysterol is chosen from the group consisting of 24-hydroxycholesterol, 25-hydroxycholesterol, 27-hydroxycholesterol, 7alpha-hydroxycholesterol, 7beta-hydroxycholesterol, 7,25-di hydroxycholesterol and 7-ketocholesterol.

A person skilled in the art will be aware of a large variety of such compounds as carriers, diluents or excipients suitable for formulating a pharmaceutical composition.

The compounds of the invention, together with conventionally used adjuvants, carriers, diluents or excipients, can be prepared in the form of pharmaceutical compositions and their unit dosages, and in said form can be used as solids, as filled tablets or capsules, or as liquids, as solutions, suspensions, emulsions, elisirs or capsules filled with the same, all for oral use, or in the form of injectable sterile solutions for parenteral administration, comprising subcutaneous and intravenous administration. Said pharmaceutical compositions and their unit dosage forms can comprise ingredients in conventional proportions, with or without other compounds or active ingredients, and said unit dosage forms can contain any suitable effective quantity of the active ingredient in proportion to the scheduled daily dosage range to be used.

The pharmaceutical compositions containing a compound of the present invention can be prepared in a manner well known in pharmaceutical technology, and comprise at least one active compound. Generally, the compounds of the present invention are administered in a pharmaceutically effective quantity. The quantity of the compound actually administered will be typically determined by a doctor, according to the circumstances, comprising the condition to be treated, the chosen method of administration, the effective compound administered, the age, weight and response of the individual patient, the gravity of the patient's symptoms and similar.

The pharmaceutical compositions of the present invention can be administered by means of a variety of methods, comprising oral, rectal, transdermal, subcutaneous, intravenous, intramuscular, intranasal and pulmonary administration. The compositions for oral administration can take the form of liquid master solutions or suspensions or master powders. More commonly, however, the compositions are presented in unit dosage forms to facilitate precise dosing. The expression "unit dosage forms" refers to physically separate units suitable as unit dosages for humans and other mammals, each unit containing a predetermined quantity of active substance calculated to produce the desired therapeutic effect, together with a suitable pharmaceutical excipient. Typical unit dosage forms comprise pre-filled pre-measured vials or syringes of liquid compositions, or pills, tablets, capsules or similar in the case of solid compositions.

Liquid forms suitable for oral administration can comprise a suitable aqueous or non-aqueous carrier with buffers, suspension and delivery agents, colourings, flavourings and similar. Solid forms can comprise, for example, any of the following ingredients or compounds of similar nature: a binder, like microcrystalline cellulose, tragacanth or gelatin; an excipient like starch or lactose, a disintegrating agent like alginic acid, Primogel or maize starch; a lubricant like magnesium stearate; a fluidifier like colloidal silicon dioxide; a sweetening agent like sucrose or saccharin; or a flavouring agent like peppermint, methyl salicylate or orange flavouring. The injectable compositions are typically based on an injectable sterile saline solution or a saline solution buffered with phosphate or other injectable carriers known in the art.

The pharmaceutical compositions can be in the form of tablets, pills, capsules, solutions, suspensions, emulsions, powders, suppositories and sustained release formulations.

If desired, the tablets can be coated by means of standard aqueous or non-aqueous techniques. In certain embodiments, said compositions and preparations can contain at least 0.1 percent of active compound. The percentage of active compound in these compositions can be varied, naturally, and can be expediently between approximately 1 percent and approximately 60 percent of the weight of the unit. The quantity of active compound in said therapeutically useful compositions is such that the therapeutically active dosage will be obtained. The active compounds can also be administered intranasally, for example as liquid drops or by sprays.

The tablets, pills, capsules and similar can also contain a binder like tragacanth, acacia gum, maize starch or gelatin; excipients like dicalcium phosphate; a disintegrating agent like maize starch, potato starch, alginic acid; a lubricant like magnesium stearate; and a sweetening agent like sucrose, lactose or saccharin. When the unit dosage form is a capsule, it can contain, in addition to the substances of the above-mentioned type, a liquid carrier like fatty oil. Various other materials can be present, as coatings or to modify the physical form of the dosage unit. For example, the tablets can be coated with shellac, sugar or both. A syrup or an elisir, in addition to the active ingredient, can contain sucrose as a sweetening agent, methylparaben and propylparaben as preservatives, a colorant and a flavouring agent like cherry or orange flavour. To prevent break-up during transit through the upper portion of the gastrointestinal tract, the composition can be a coated enteric formulation.

The compositions for topical administration comprise, but are not limited to, ointments, creams, lotions, solutions, pastes, gels, sticks, liposomes, nanoparticles, patches, bindings and bandages for wounds. In certain embodiments, the topical formulation comprises a penetration improver.

The compositions for pulmonary administration comprise, but are not limited to, compositions of anhydrous powders consisting of the powder of a compound having formula (I) or a salt thereof, and the powder of a suitable carrier and/or lubricant. The compositions for pulmonary administration can be inhaled by any suitable dry powder inhaler known to a person skilled in the art.

The compositions are administered according to a protocol and a dosage sufficient to reduce the patient's inflammation and pain. In some embodiments, in the pharmaceutical compositions of the present invention the active ingredient or active ingredients are generally formulated in dosage units. The dosage unit can contain 0.1 to 1,000 mg of a compound having formula (I) per dosage unit for daily administration.

In some embodiments, the effective quantities for a topical formulation will depend on the gravity of the disease, the disorder or the condition prior to the therapy, the state of health of the individual and the response to the drug. In some embodiments, the dose ranges from 0.001% by weight to approximately 60% by weight of the formulation.

When used in combination with one or more other active ingredients, the compound of the present invention and the other active ingredient can be used at lower doses than when each one is used individually.

Referring to formulations and any variety of administration routes, methods and formulations for the administration of drugs are described in Remington's Pharmaceutical Sciences, 17th Edition, Gennaro et al. Eds., Mack Publishing Co., 1985*, and* Remington's Pharmaceutical Sciences, Gennaro AR ed. 20th Edition, 2000, Williams & Wilkins PA, USA*, and* Remington: The Science and Practice of Pharmacy, 21st Edition, Lippincott Williams & Wilkins Eds. , 2005*;* and in Ansel's Pharmaceutical Dosage Forms and Drug Delivery Systems, 8th Edition, Lippincott Williams & Wilkins Eds., 2005.

The above-described components for injectable compositions or oral administration are purely representative.

The compounds of the present invention can also be administered in sustained release forms or by systems for the administration of drugs with sustained release.

Further characteristics of the present invention will become clear from the following description of some merely illustrative non-limiting examples.

### MATERIALS AND METHODS

### CELL LINES AND VIRUSES

The cell line 293TT, derived from human embryonic renal cells transformed with a plasmid vector codifying the Simian virus 40 (SV40) large T antigen, was cultivated as a monolayer with Dulbecco's modified Eagle's medium (DMEM) (Gibco-BRL, Gaithersburg, MD) integrated with 10% heat inactivated fetal bovine serum (FBS), Glutamax-I (Invitrogen, Carlsbad, CA) and non-essential amino acids. The Siha, C33a and HeLa cells of human epithelial adenocarcinoma (ATCC® CCL-2™), African green monkey kidney epithelial cells (MA104) and human colorectal adenocarcinoma epithelial cells (Caco-2) were cultivated in minimum essential medium (MEM) or in DMEM supplemented with 10% heat inactivated FBS and 1% Zell Shield, at 37°C in a 5% CO₂ atmosphere.

The strains of human rotavirus Wa (ATCC® VR-2018), WI61 (ATCC® VR-2551), HRV 408 (ATCC® VR-2273), HRV 248 (ATCC® VR-2274) and DS-1 (ATCC® VR-2550) were acquired from the American Tissue Culture Collection (ATCC); the viruses were activated with 5µg/ml porcine pancreatic trypsin of type IX (Sigma, St. Louis, Mo) for 30 minutes at 37°C and propagated in MA104 cells using MEM containing 0.5 µg of trypsin per ml as described previously (27).

The human rhinovirus 1A (ATCC® VR-1559) was acquired from ATCC. The virus was propagated in HeLa cells, at 33°C with 5% of CO₂. When the cytopathic effect (CPE) had fully developed, cells and supernatants were collected, frozen and thawed three times, clarified and divided into aliquots. The viruses were kept at -70°C. The rhinovirus titres were determined by means of the standard plaque assay. In short, the HeLa cells were seeded 2 days prior to the infection in plates with 96 wells, reaching 60%-70% confluence at the time of the infection. The viral suspension was serially diluted in DMEM supplemented with 2% fetal bovine serum and inoculated; the infected wells were incubated at 33°C for 1 hour, allowing the viruses to adhere and enter the cells. The cells were subsequently washed with medium and covered with a 1:1 mixture of 1.6% SeaPlaque agarose (BioWhittaker Molecular Applications) and DMEM 2X [26.76g DMEM (Gibco BRL), 20 ml of L-glutamine (Gibco BRL), 4.4g of sodium bicarbonate (Sigma, St. Louis, Mo), FBS 40ml (Sigma, St. Louis, Mo) and 1000 ml of water]. The plates were incubated at 33°C for 3 days. At the end of the incubation, the plates were fixed with 7.5% formaldehyde (Fluka) and coloured with crystal violet (Sigma, St. Louis, Mo). The number of plaques formed was then counted; the virus titres were expressed in terms of plaque-forming units per ml (PFU/ml).

### PRODUCTION OF HPV PsV

The plasmids and cells 293TT used for the production of pseudovirus (PsV) were kindly supplied by John Schiller (National Cancer Institute, Bethesda, MD). The detailed protocols and the plasmid maps can be found on the website: http://home.ccr.cancer.gov/lco/default.asp. The HPV-16 PsV were produced according to the method previously described. In short, the cells 293TT were cotransfected with plasmids that express the major and minor capsid proteins (L1 and L2, respectively) of papillomavirus, together with a reporter plasmid that expresses GFP. The HPV-16 PsV were produced using bicistronic expression plasmids L1/L2 (p16sheLL). The capsids were left to mature overnight in cell lysate; the clarified supernatant was then loaded in a density gradient between 27 and 39% of OptiPrep (Sigma-Aldrich, St. Louis, MO) at ambient temperature for 3 hours. The material was centrifuged at 28000 rpm for 16 hours at 4°C in a SW41.1 rotor (Beckman Coulter, Inc., Fullerton, CA) and then collected by means of a puncture on the bottom of the test tubes. The fractions were inspected for purity in 10% acrylamide gel and tris-glycine sodium dodecylsulfate (SDS), titred on cells 293TT to verify infectivity by means of GFP detection, and then frozen at - 80°C until the time of use. The protein L1 content of the PsV produced was determined by comparison with bovine serum albumin standard in SDS-polyacrylamide gel coloured with Coomassie.

### CELL VIABILITY ASSAY

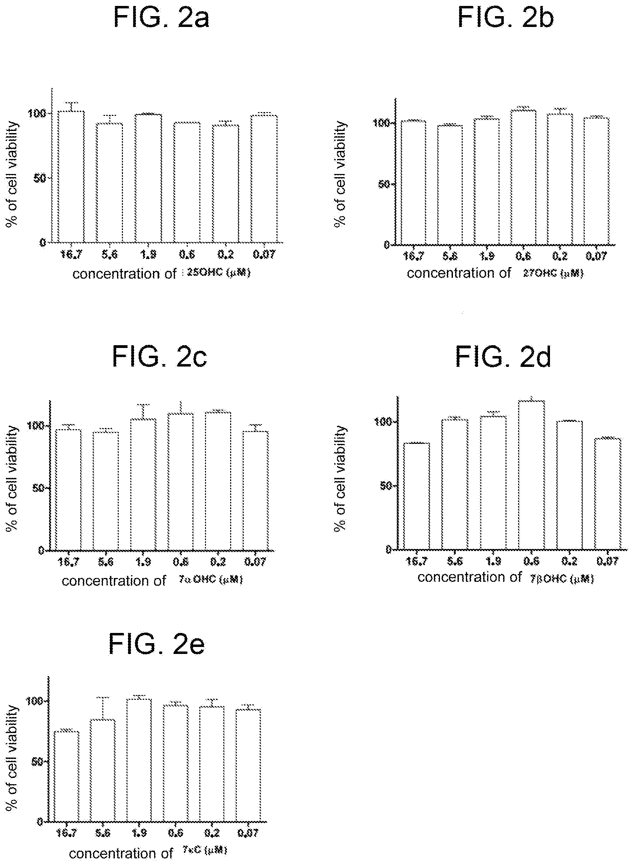

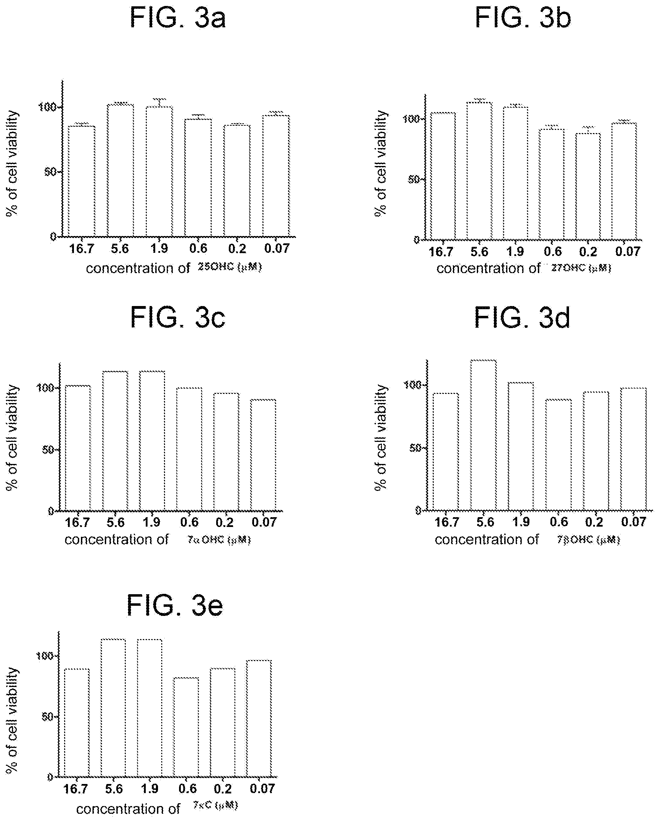

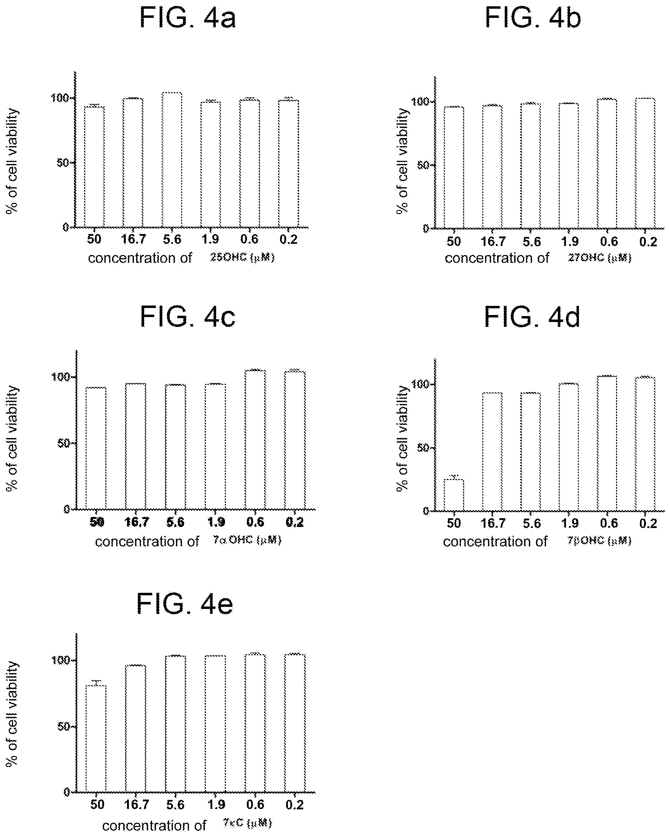

The cells were seeded at a density of 5 x 10³/well in plates with 96 wells and treated the following day with 25OHC, 27OHC, 7αOHC, 7βOHC and 7κC at various dilutions using as a control wells incubated with equal volume of ethanol (the solvent used for the oxysterols). After 24 hours of incubation, the cell viability was determined using the CellTiter 96 Proliferation Assay kit (Promega, Madison, WI, USA), according to the manufacturer's instructions. The absorbances were measured at a wavelength of 490nm, using a plate reader (Model 680, BIORAD). The effect on the cell viability of oxysterols at different concentrations was expressed as a percentage, comparing the absorbances of the samples treated with the respective controls. The results on the cells are reported in figures 2a-2e (MA104 cells), in figures 3a-3e (CaCo2 cells) and in figures 4a-4e (Hela cells). From these experiments it emerges that the concentrations at which the oxysterols show an antiviral activity are not cytotoxic.

### INFECTIVITY ASSAY FOR ROTAVIRUS

The antiviral effectiveness of the oxysterols of the invention was determined by means of infectivity inhibition assay. The assays were performed with confluent monolayers of MA104 cells seeded in plates with 96 wells, as described previously (28). The cells were treated for 20 hours at 37°C with the 25OHC, 27OHC or the 24OHC at concentrations between 0.07 and 16.7 µM. The control samples were prepared by treating the cells with culture medium with the addition of equal volumes of ethanol. The cells were then washed with medium, and the rotavirus infection was performed at a multiplicity of infection (MOI) of 0.02. After the infection, the cells were washed with medium and incubated at 37°C in an incubator humidified at 5% (vol/vol) CO₂-95% (vol/vol) of air. After 16 hours, the infected cells were fixed with a cold acetone-methanol mixture (50:50), and the virus titres were determined by means of indirect immunocytochemistry. The inhibition of the virus infectivity was expressed as a percentage comparing the mean number of infective events in the treated and non-treated samples ± the standard deviations. Where possible, the values of the oxysterol concentrations able to determine 50% or 90% of the antiviral effect (EC₅₀ and EC₉₀ respectively) were calculated by means of regression analysis using the dose-response curves generated by the experimental data, with the use of PRISM 5.00 (GraphPad Software, San Diego, California, USA). The results obtained on the MA104 cells are reported in Table 1.

**Table 1**

| Rotavirus strain | 25OHC | | 27OHC | | 24OHC | |
|---|---|---|---|---|---|---|
| | EC50 (*µ*M) | EC90 (*µ*M) | EC50 (*µ*M) | EC90 (*µ*M) | EC50 (*µ*M) | EC90 (*µ*M) |
| Wa | 0.16 | 1.37 | 0.26 | 1.73 | 0.39 | 1.6 |
| HRV 248 | 0.11 | 1.26 | 0.23 | 3.84 | n.a.* | n.a. |
| DS-1 | 0.27 | 5.77 | 0.72 | 5.85 | n.a. | n.a. |
| WI61 | 0.09 | 0.28 | 0.19 | 0.74 | n.a. | n.a. |
| HRV 408 | 0.12 | 0.42 | 0.4 | 4.56 | n.a. | n.a. |

| | | | | | | |
|---|---|---|---|---|---|---|
| *n.a. not assayed | | | | | | |

The results of the antiviral assays performed treating with 25OHC and 27OHC were confirmed repeating the infectivity assays on Caco2 cells. The data thus obtained are reported in Table 2.

**Table 2**

| Rotavirus strain | 25OHC | | 27OHC | |
|---|---|---|---|---|
| | EC50 (*µ*M) | EC90 (*µ*M) | EC50 (*µ*M) | EC90 (*µ*M) |
| Wa | 0.19 | 1.26 | 0.44 | 2.50 |

The results show that the 25OHC and the 270HC both have antiviral activity vis-à-vis the rotavirus; said effectiveness is not limited to one single cell model or to one single human rotavirus strain. Furthermore, also an oxysterol of enzymatic origin present at cerebral tissue level, i.e. the 24OHC, has antiviral activity vis-à-vis the human rotavirus.

### ROTAVIRUS YIELD REDUCTION ASSAY

To verify the ability of the oxysterols of the invention to inhibit several viral replication cycles, the MA104 cells were seeded in plates with 24 wells, until reaching confluence. The cells were treated at 37°C with 25-, 27-, 7α-, 7β-hydroxycholesterol or 7-ketocholesterol at 5.6 µM for 20 hours, then washed with medium and infected with the human rotavirus at an MOI of 0.02 (strain Wa) activated with trypsin. After one hour, the cells were washed and fresh medium was added supplemented with 0.5 µg/ml of porcine trypsin. The infected cells and the supernatants were collected 48 hours after the infection and quantified. The assay was performed in triplicate and the results were confirmed on Caco2 cells. In an alternative protocol, the MA104 cells were treated with oxysterols - at the concentration indicated above - after the viral inoculum. The one-way variance analysis (ANOVA), followed by the Bonferroni test, was used to evaluate the statistical significance of the virus titre differences between the treated and non-treated samples. The significance was fixed at 95%.

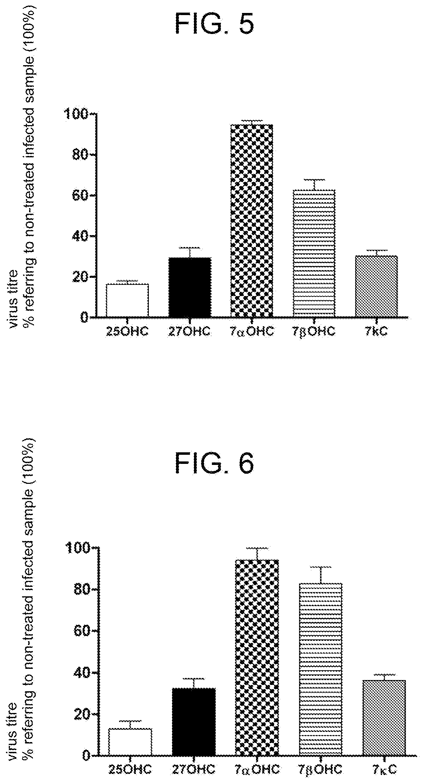

The results of the pre-treatment assays are illustrated in figures 5 (MA104 cells) and 6 (Caco2 cells).

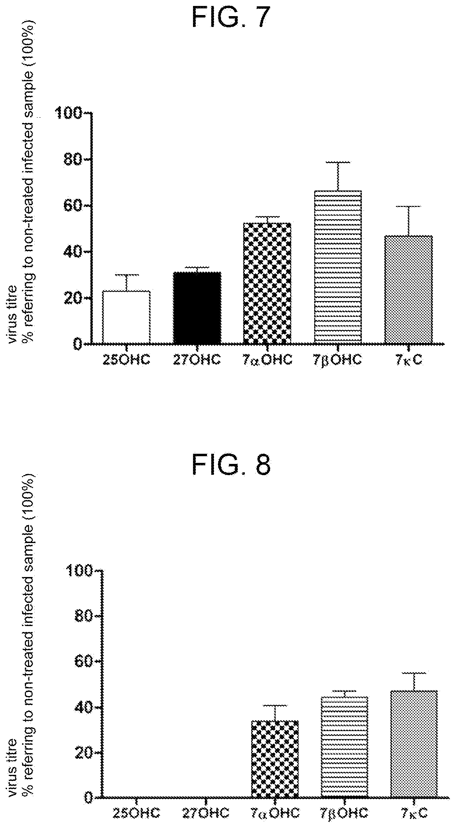

25OHC, 27OHC and 7κC significantly limit (p <0.05) the production of virus progenies in both the cell lines analysed. 7βOHC shows a significant (P<0.05) antiviral activity only on the MA104 cells. The results of the post-infection treatment assays confirm the in vitro effectiveness of the 25OHC and 27OHC, and highlight the antiviral activity also of the oxysterols 7αOHC, 7βOHC and 7κC (figure 7). It should be noted, however, that the entity of the inhibition induced by 7αOHC, 7βOHC and 7KC is significantly lower than that of the 25OHC and 27OHC.

### INFECTIVITY ASSAY WITH RHINOVIRUS

The HeLa cells, kept in DMEM supplemented with 10% FBS and 1% Zell Shield, were seeded (at 8 × 10⁴ cells/well) in plates with 24 wells. The medium was removed and the cells were infected with 200 microlitres/well containing approximately 30 PFU of a stock of human rhinovirus 1A. The infected cells were incubated at 33°C for 1 h, allowing the virus to adhere and enter the cells. After the incubation, the cells were washed with medium, and covered with a 1:1 solution of SeaPlaque agarose at 1.6% and DMEM 2X containing different concentrations of 25OHC and 27OHC (5.6 µM to 0.07 µM). In an alternative protocol, the cells were plated and treated with the same scalar concentrations of 25OHC, 27OHC or 24OHC for 20 hours prior to the infection. The plates were incubated at 33°C for 5 days. After the incubation, the plates were fixed with formaldehyde at 7.5% (Fluka), coloured with crystal violet (Sigma, St. Louis, Mo) and the number of plaques formed was counted. The oxysterol concentration values able to determine 50% or 90% of the antiviral effect (EC₅₀ and EC₉₀ respectively) were calculated by means of regression analysis using the dose-response curves generated by the experimental data, using PRISM 5.00 (GraphPad Software, San Diego, California, USA). These results are illustrated in tables 3 and 4, for the post-infection treatment assay and for the pre-treatment assay respectively.

**Table 3**

| Oxysterol | EC50 (µM) | EC90 (µM) |
|---|---|---|
| 25OHC | 0.16 | 0.41 |
| 27OHC | 0.48 | 1.27 |

**Table 4**

| Oxysterol | EC50 (µM) | EC90 (µM) |
|---|---|---|
| 25OHC | 0.29 | 0.54 |
| 27OHC | 0.62 | 2.17 |
| 24OHC | 0.77 | 3.10 |

The results show that treatment of the cells with 25OHC, 27OHC or 24OHC is not only able to reduce the susceptibility of the cells to the infection with rhinovirus, but is also able to inhibit replication after the inoculum.

### RHINOVIRUS YIELD REDUCTION ASSAY

To verify the ability of the oxysterols of the invention to limit the production of viral progenies, the result of multiple replication cycles, the HeLa cells were seeded in plates with 24 wells. Each well was inoculated with 200 µl of a stock of human rhinovirus 1A containing approximately 30 PFU per well and incubated for 1 hour. After the incubation, the cells were washed with medium, and fresh medium was added (DMEM supplemented with 2% FBS) containing the oxysterols 25OHC, 27OHC, 7αOHC, 7βOHC and 7κC at a concentration of 5.6µM. The infected cells and the supernatants were collected 5 days after the infection (when a complete CPE was visible), and titred by means of the standard plaque method, as described above. The results are illustrated in figure 8: the treatment with 25OHC and 27OHC is able to totally inhibit the production of viral progenies, while the remaining oxysterols, although able to significantly limit (p<0.05) the production of progenies, have a lower effectiveness.

### NEUTRALIZATION ASSAYS WITH HPV PSEUDOVIRIONS

HeLa cells (or C33A or Siha cells) were seeded 24 hours beforehand in flat-bottomed plates with 96 wells at a density of 8000 cells/well in 100 µl. The following day, the cells were treated with serial dilutions of 25OHC and 27OHC. After 16 hours the oxysterols were removed and PsV of HPV-16 were added at a dose corresponding to approximately 1ng/ml of L1. The mixture was incubated for 72 hours at 37°C and the GFP-expressing infected cells were observed with an inverted fluorescence microscope Zeiss LSM510 (Zeiss, Oberkochen, Germany). The results on the Hela cells are illustrated in table 5: 25OHC, 27OHC and 24OHC show antiviral activity vis-à-vis HPV, and are characterised by EC50 in micromoles. 25OHC proved more effective than 27OHC and 24OHC.

**Table 5**

| Oxysterol | Cell line | EC50 (*µ*M) |
|---|---|---|
| 25OHC | HeLa | 2.19 |
| 27OHC | HeLa | 9.33 |
| 24OHC | HeLa | 4.38 |

The EC50 values of the 25OHC and 27OHC measured on the other cell lines are reported in Table 6.

**Table 6**

| Oxysterol | Cell line | EC50 (*µ*M) |
|---|---|---|
| 25OHC | SiHa | 14.75 |
| | C33A | 2.06 |
| 27OHC | SiHa | 28.87 |
| | C33A | 6.87 |

25OHC and 27OHC show inhibitory activity also in SiHa cells and C33A cells, therefore their inhibitory activity is not limited to one single cell model.

### QUANTITATIVE REAL TIME RT-PCR

The Caco2 cells were cultivated in DMEM medium containing 10% serum and seeded in 25 cm² flasks (8 million confluence cells). The next day the cells were treated, in DMEM medium containing 2% serum, with 1.9 *µ*M 25OHC or 27OHC (dissolved in ethanol) for 30 minutes, 1, 2, 3, 4, 5 or 6 hours. Extraction of the total RNA was performed using the TRIzol Reagent (Applied Biosystems, Monza, Italy) and the quantity and purity (A260/A280 ratio) of the extracted RNA were spectrophotometrically measured. Subsequently, 2 *µ*g of RNA were retrotranscribed using the High-Capacity cDNA Reverse Transcription Kit (Applied Biosystems). Quantitative RT-PCR was performed with 30 ng of cDNA using the "TaqMan Gene Expression Assay kits" for TLR-3 and β-actin, TaqMan Fast Universal PCR Master Mix, and 7500 Fast Real-Time PCR System (Applied Biosystems). For each gene considered, the threshold cycle (Ct) was determined and the results were normalized using β-actin as a reference gene. The relative quantification was performed using a mathematical method previously published (29). In figures 1a-1b, the results are reported as increment of the genic expression (Fold change, Fc) of the treated samples with respect to the controls.

### STATISTICAL ANALYSIS

The results obtained in the experiments on infectivity inhibition and viral yield in the presence and absence of oxysterols were subjected to variance analysis (ANOVA) followed by a Bonferroni post-test, using GraphPad Prism 5.00 (GraphPad Software).

### MECHANISM OF ACTION

### ASSAY ON ADHESION OF THE ROTAVIRUS TO THE CELL SURFACE.

For the assays on bonding of the rotavirus to the surface of MA104 cells, monolayers of confluent cells in plates with 24 wells were treated at 37°C with 25- or 27-hydroxycholesterol at 1.9 µM and 5.6 µM for 20 hours. The cells were then washed and cooled on ice for 20 minutes. The infectivity of the virus was activated with 5 µg of porcine trypsin (Sigma)/ml for 30 minutes at 37°C. The activated virus, which had been cooled to 4°C, was left to adhere to the cells in ice for 1 hour at an MOI of 3. Lastly, the cells were washed with cold MEM; cold MEM was then added to the cells, which underwent two freezing-thawing cycles, in order to allow detachment of the viral particles bonded to the cell surface; the cell lysate was incubated at 37°C for 30 minutes in the presence of 10 mg of porcine trypsin/ml, subsequently clarified by means of centrifugation (1200 rpm, 10 minutes).

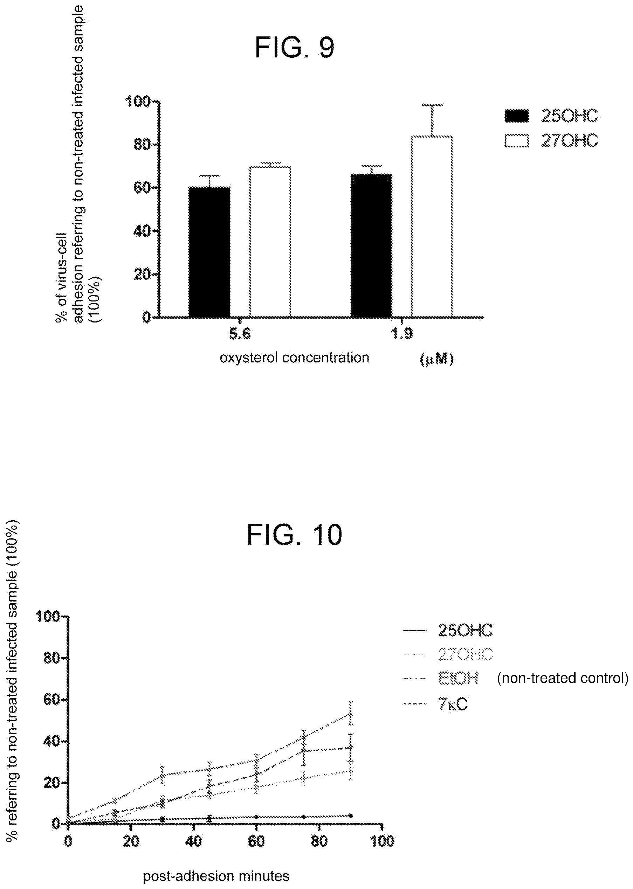

The titre of the virus population bound to the cells was determined by means of indirect immunocytochemistry performed on monolayers of MA104 cells infected with serial dilutions of the virus collected. The results are illustrated by figure 9; in the graph, the titre of the viral population bound to the surface of the treated cells is expressed in percentage terms with respect to the non-treated control and the error bars represent the standard deviation. To evaluate the statistical significance of the differences in the virus-cell bond, the two-way ANOVA test was used, followed by the Bonferroni test. The significance was fixed at the level of 95%. The results show that only the 25OHC (at a dose of 5.6 µM) is able to reduce in a statistically significant manner (p<0.05) the ability of the virus to adhere to the cell surface.

### ROTAVIRUS CELL ENTRY KINETICS

To measure the entry speed of the rotavirus into the cells, confluent monolayers of MA104 cells (cultivated in plates with 96 wells) were treated with 25OHC, 27OHC or 7KC at 1.9 µM at 37°C; after 20 hours, the cells were washed extensively with cold medium and left to cool on ice. The activated virus, which had been cooled to 4°C, was left to adhere to the cells in ice for 1 hour at a multiplicity of infection (MOI) of 0.02. The non-bound viruses were then eliminated by washing, medium was added at 37°C, and the plates were incubated at 37°C in a humidified atmosphere. At fixed times (i.e. 0, 15, 30, 45, 60, 75 and 90 minutes after the virus-cell adhesion), the medium was extracted from the wells and the viral particles still present on the surface of the cells were eliminated by means of two rapid washing operations with EGTA 3 mM in PBS, followed by incubation in warm medium. After reaching the last time point (90 minutes from the virus-cell adhesion), the plates with 96 wells were placed in an incubator (37°C, 5%CO2), and the infection was left to proceed for a further 16 hours. The cells were then fixed and coloured as described above. The quantity of viral particles that entered the cells at each time point was compared with the quantity of virus that entered when no washing with EGTA was performed (100% entry). The ANOVA, followed by the Bonferroni test, was used to evaluate the statistical significance of the differences between the treated and non-treated samples. The significance was fixed at the level of 95%.

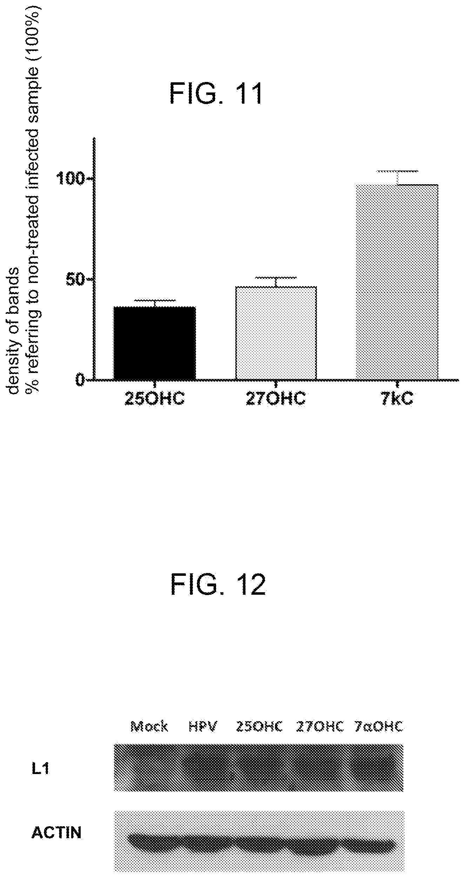

The results are illustrated in figure 10, and are expressed as a percentage, comparing treated cells with cells incubated with culture medium: the treatment with 25OHC is able to totally inhibit the ability of the rotavirus to penetrate into the host cells; the 27OHC, although able to limit the entry of the viral particles into the cells, shows a lesser effectiveness. Lastly, the treatment with 7κC does not cause a significant reduction (p>0.05) in the number of infective events. As a control experiment, the same treatment and infection protocol was performed on MA104 cells, cultivated in a plate with 6 wells. In this experiment, after reaching the last time point of the entry measurement (90 minutes), the cells were lysed, the proteins extracted were denatured by boiling for 5 minutes, then separated by electrophoresis on sodium dodecylsulfate gel with 12% polyacrylamide (SDS-12%PAGE) and transferred to a polyvinylidene difluoride (PVDF) membrane. The protein of the intermediate capsid VP6 was detected using the murine monoclonal antibody 2B4 (mab0036-P, CovalAb) at a dilution 1:750, followed by secondary antibody IgG-HRP (Santa Cruz Biotechnology Inc.). The actin was detected using the anti-actin murine monoclonal antibody MAB1501R (Millipore), followed by antibody IgG-HRP (Santa Cruz Biotechnology Inc.). The density of the bands was compared using the ImageJ software, and the intensity of the actin bands was used to normalize the values. The ANOVA, followed by the Bonferroni test, was used to evaluate the statistical significance of the differences between the treated and the non-treated samples. The significance was fixed at the level of 95%. The results are reported in figure 11 and are expressed as a percentage, comparing treated cells with cells incubated with culture medium. The method allowed location - within the cells treated with 25OHC and 27OHC - of the viral antigen, in a significantly (p<0.05) lower quantity than the non-treated control; this fact confirms the ability of the two molecules to reduce the rotavirus entry rate into the host cell.

### ASSAY FOR ADHESION OF THE PAPILLOMAVIRUS TO THE CELL SURFACE: WESTERN BLOT ANALYSIS.

The Hela cells were treated for 16 hours with 25OHC, 27OHC and 7αOHC (the latter used as a negative control, since it is not able to inhibit the infectivity of HPV) and subsequently incubated for 4 hours at 4°C with the PsV; the cells were then lysed and the proteins extracted were separated by means of SDS-PAGE and transferred to a polyvinylidene difluoride (PVDF) membrane. L1 was detected using the murine monoclonal antibody (Ab30908, Abcam, Cambridge, UK) at a dilution of 1:2000, followed by secondary antibody IgG-HRP (Santa Cruz Biotechnology Inc.). The actin was detected using an anti-actin murine monoclonal antibody (MAB1501R, Millipore), followed by secondary antibody IgG-HRP (Santa Cruz Biotechnology Inc.).

The results obtained are shown in figure 12. It can be seen that 25OHC and 27OHC are not able to inhibit adhesion of the PsV to the cell surface: the protein L1 of HPV can be located on the surface of both treated and non-treated cells, therefore the inhibition mediated by 25OHC and 27OHC occurs in a phase subsequent to the cell adhesion. The pseudovirions of papillomavirus used in the experiments are not able to replicate in the host cell, but only adhere to the host cell and penetrate inside it; since the ability of the pseudovirions to adhere to the surface of the host cell is not inhibited by the treatment with 25OHC or 27OHC, it was concluded that the inhibited replication cycle phase is the cell entry phase.

### TARGET IDENTIFICATION BY siRNA SENSITIZATION (TISS) ASSAYS

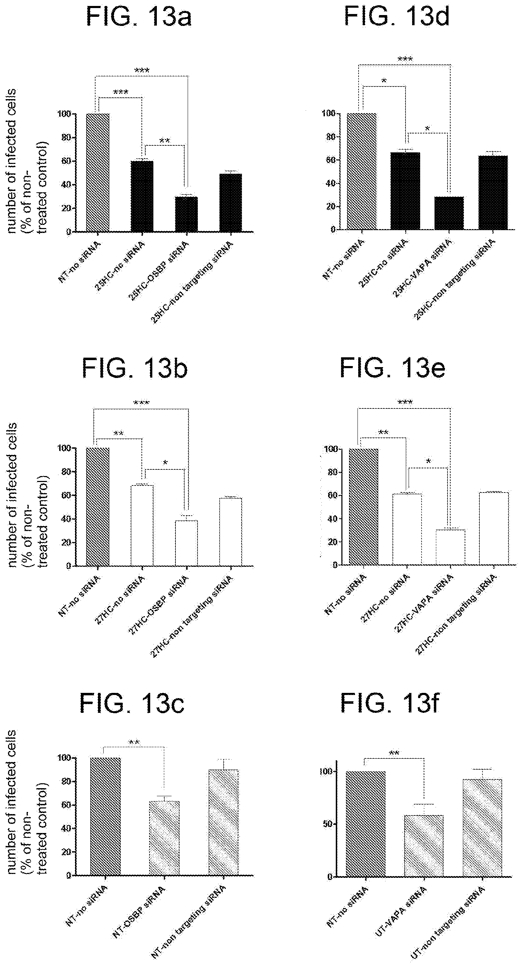

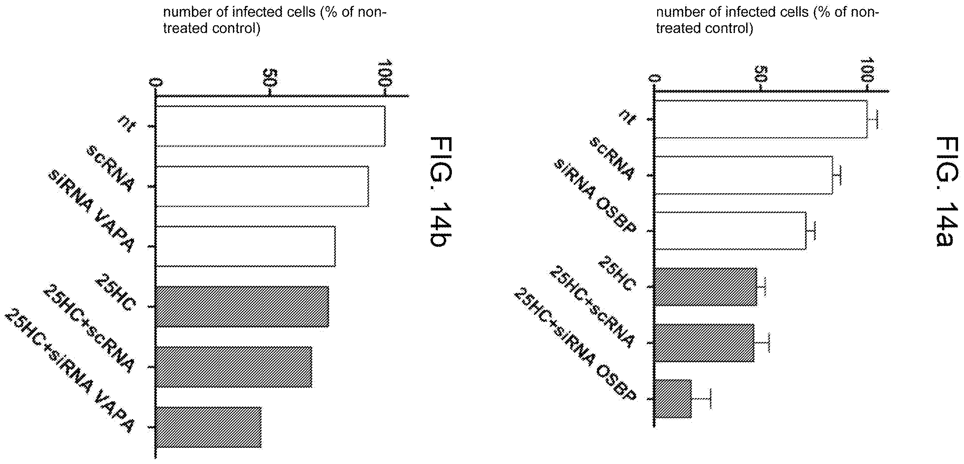

After identifying entry into the host cell as the rotavirus and papillomavirus replication cycle phase inhibited by the oxysterols, we evaluated what the cell targets of the 250HC and the 270HC responsible for their antiviral activity were. In this regard, it was hypothesised that two proteins, vesicle-associated membrane protein-associated protein A (VAPA) and the oxysterol binding protein (OSBP) were involved in the mechanism of action of the oxysterols assayed. VAPA and OSBP regulate intracellular cholesterol homeostasis, which is fundamental for the replication of numerous viruses, both naked and enveloped. During entry, the viruses or the vesicles containing the viruses can fuse with the membranes of the endosomes to mediate release of the virions into the cytosol, and an alteration in the quantity of endosomal cholesterol can inhibit this phase of the replication cycle. In this regard, it has been shown that the transmembrane protein 3 induced by interferon (IFITM3) interacts with VAPA inhibiting the interaction with OSBP, altering the homeostasis of the intracellular cholesterol and inhibiting entry of the virus into the host cell (30). By altering the functions of the VAPA-OSBP complex, IFITM3 induces a significant accumulation of cholesterol on the membrane of the late endosomes, trapping the virus inside these vesicles and inhibiting entry into the host cell. Since the oxysterols are the main interactors of OSBP and since they are able to inhibit entry of the rotavirus and papillomavirus into the host cell, it has been hypothesised that they act analogously to IFITM3, i.e. inhibiting the interaction between OSBP and VAPA. This mechanism of action vis-à-vis the rotavirus was therefore investigated, performing a target identification assay by means of sensitization with short interfering RNA (siRNA). The Caco2 cells (for the experiments with human rotavirus) or the HeLa cells (for the experiments with human papillomavirus) were seeded in a plate with 24 wells; during seeding, the cells were transfected with siRNA specific for OSBP and VAPA (at a concentration of 20nM). After 72 hours, the medium containing the siRNA was removed, and the cells were treated with 25OHC or 27OHC at concentrations equal to the respective EC50. After one day, the medium was removed and the cells inoculated with the respective viruses in the same time, temperature and MOI conditions described in the protocols of the infectivity or neutralization assays. The number of infected cells was evaluated as described in the preceding paragraphs. The results illustrated in figures 13 and 14 show that, in cells in which the expression of OSBP and VAPA has been silenced, the infectivity of the human rotavirus and papillomavirus is partially inhibited.

In particular figure 13 illustrates the effect of the silencing of OSBP and VAP-A on the infectivity of the human rotavirus. The antiviral activity of the 25OHC and the 27OHC is higher in cells in which the expression of OSBP (25HC OSBP-siRNA; 27HC OSBP-siRNA) or VAP-A (25HC VAPA-siRNA; 27HC VAPA-siRNA) has been silenced (panels A, B, D, E). Furthermore, the sole silencing of OSBP or VAP-A (NT-OSBP siRNA and NT-VAPA siRNA) causes inhibition of the infectivity of the human rotavirus (panels C and F) with respect to the non-treated control (NT-no siRNA).

Figure 14 illustrates the effect of the silencing of OSBP and VAP-A on the infectivity of the human papillomavirus. The antiviral activity of the 25OHC is higher in cells in which the expression of OSBP (25HC+siRNA OSBP) or VAP-A (25HC+siRNA VAPA) has been silenced (panels A and B). Furthermore, the sole silencing of OSBP or VAP-A (siRNA OSBP and siRNA VAPA) causes inhibition of the infectivity of the human papillomavirus (panels A and B) with respect to the non-treated control (nt).

Furthermore, the transfection is able to improve the in vitro effectiveness of the 25OHC vis-à-vis the papillomavirus and of the 25OHC and 27OHC vis-à-vis the rotavirus, making the cells more sensitive to the treatment.

### ANTIVIRAL ASSAYS WITH U18666A

According to the hypothesis formulated previously, the oxysterols determine an accumulation of the intracellular cholesterol at endosomal level, inhibiting entry of the rotavirus. In order to consolidate this hypothesis, the antiviral activity of the compound U18666A (VWR INTERNATIONAL PBI SRL) was evaluated vis-à-vis the human rotavirus and papillomavirus. U18666A is an amphipathic steroid, widely used to block the intracellular transport of cholesterol, with consequent accumulation of the same at the level of the endosome membranes (31). In short, antiviral assays were performed treating the MA104 cells (for the rotavirus) or HeLa cells (for the papillomavirus) for 20 hours with scalar concentrations of U18666A (0.07 to 50*µ*M) and then infecting for one hour with the respective viruses. The effect of the treatment on the infectivity of the two viral models was evaluated as described previously. The results show that U18666A has antiviral activity vis-à-vis the human papillomavirus, and has an EC50 in micromoles (Table 7). The experiment described therefore constitutes solid proof of the principle of the mechanism of action proposed.

**Table 7**

| | EC50 (*µ*M) |
|---|---|
| U18666A | 1.74 |

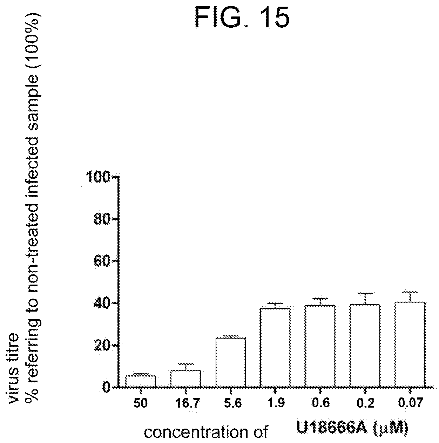

Also vis-à-vis the human rotavirus, the treatment with U18666A determines inhibition of the infectivity; in these experiments, the antiviral activity is so high that calculation of the EC50 is not possible. The results are therefore illustrated in figure 15. Overall, these data confirm the hypothesis formulated initially, demonstrating that U18666A, a molecule whose effect on cholesterol cell homeostasis is known in literature, has an antiviral activity vis-à-vis the rotavirus and papillomavirus.

On the basis of the results obtained, it can be concluded that the antiviral activity of the 25OHC and 27HC is linked to their ability to alter the intracellular homeostasis of the cholesterol (but without compromising the cell viability), interfering with the signalling route mediated by VAPA and OSBP and blocking entry of the rotavirus and papillomavirus into the host cell. These results therefore propose a completely new mechanism of action of the oxysterols vis-à-vis the naked viruses. For example, Roulin and colleagues (32) have shown that the 25OHC is able to inhibit replication of the genoma of a naked virus (the rhinovirus) but not entry into the host cell. These data show that the oxysterols have different mechanisms of action vis-à-vis different virus families; said affirmation is particularly important if contextualised in the current panorama of research into the antivirals and antiviral drugs on the market: at the moment, molecules with a wide spectrum of activity (i.e. active vis-à-vis both enveloped and naked viruses) and with different mechanisms of action vis-à-vis different viruses do not exist.

### REFERENCES

1. Glass RI, Parashar UD, Bresee JS, Turcios R, Fischer TK, Widdowson MA, Jiang B, Gentsch JR. (2006) Rotavirus vaccines: current prospects and future challenges. Lancet 368: 323-332.
2. Tate JE, Burton AH, Boschi-Pinto C, Steele AD, Duque J, Parashar UD; WHO-coordinated Global Rotavirus Surveillance Network. (2012) 2008 estimate of worldwide rotavirus-associated mortality in children younger than 5 years before the introduction of universal rotavirus vaccination programmes: a systematic review and meta-analysis. Lancet Infect Dis 12: 136-141.
3. Macartney KK, Porwal M, Dalton D, Cripps T, Maldigri T, Isaacs D, Kesson A. (2011) Decline in rotavirus hospitalisations following introduction of Australia's national rotavirus immunisation programme. J Paediatr Child Health 47: 266-270.
4. Ward RL, Bernstein DI. (2009) Rotarix: a rotavirus vaccine for the world. Clin Infect Dis 48: 222-228.
5. Rollinger JM, Schmidtke M. (2009) The human rhinovirus: human-pathological impact, mechanisms of antirhinoviral agents, and strategies for their discovery Med Res Rev 31: 42-92.
6. Makela MJ, Puhakka T, Ruuskanen O, Leinonen M, Saikku P, Kimpimaki M, Blomqvist S, Hyypia T, Arstila P.(1998) Viruses and bacteria in the etiology of the common cold J Clin Microbiol 36: 539-542.
7. Arruda E, Pitkaranta A, Witek TJ Jr., Doyle CA, Hayden FG. (1997) Frequency and natural history of rhinovirus infections in adults during autumn. J Clin Microbiol 35: 2864-2868.
8. Fendrick AM, Monto AS, Nightengale B, Sarnes M. (2003) The economic burden of non-influenza-related viral respiratory tract infection in the United States. Arch Intern Med 163: 487-494.
9. Mallia P, Message SD, Gielen V, Contoli M, Gray K, Kebadze T, Aniscenko J, Laza-Stanca V, Edwards MR, Slater L, Papi A, Stanciu LA, Kon OM, Johnson M, Johnston SL. (2011) Experimental rhinovirus infection as a human model of chronic obstructive pulmonary disease exacerbation Am. J Respir Crit Care Med 183: 734-742.
10. Jackson DJ, Johnston SL. (2010) The role of viruses in acute exacerbations of asthma J Allergy Clin Immunol 125: 1178-1187.
11. Papi A, Contoli M. (2011) Rhinovirus vaccination: the case against. Eur Respir J 37: 5-7.
12. Palmenberg AC, Spiro D, Kuzmickas R, Wang S, Djikeng A, Rathe JA, Fraser-Liggett CM, Liggett SB. (2009) Sequencing and analyses of all known human rhinovirus genomes reveal structure and evolution. Science 324: 55-59.
13. Greer CE, Wheeler CM, Ladner MB, Beutner K, Coyne MY, Liang H, Langenberg A, Yen TS, Ralston R. (1995) Human papillomavirus (HPV) type distribution and serological response to HPV type 6 virus-like particles in patients with genital warts. J Clin Microbiol 33: 2058-2063.
14. Arbyn M, Castellsague X, de Sanjose S, Bruni L, Saraiya M, Bray F, Ferlay J. (2011). Worldwide burden of cervical cancer in 2008. Ann Oncol 22: 2675-2686.
15. Cogliano V, Baan R, Straif K, Grosse Y, Secretan B, El Ghissassi F, Cancer WHOIAfRo. (2005) Carcinogenicity of human papillomaviruses. Lancet Oncology 6: 204.
16. zur Hausen H. (2009) Papillomaviruses in the causation of human cancers - a brief historical account. Virology 384: 260-265.
17. Paavonen J, Jenkins D, Bosch FX, Naud P, Salmeron J, Wheeler CM, Chow SN, Apter DL, Kitchener HC, Castellsague X, de Carvalho NS, Skinner SR, Harper DM, Hedrick JA, Jaisamrarn U, Limson GA, Dionne M, Quint W, Spiessens B, Peeters P, Struyf F, Wieting SL, Lehtinen MO, Dubin G, group HPs. (2007) Efficacy of a prophylactic adjuvanted bivalent L1 virus-like-particle vaccine against infection with human papillomavirus types 16 and 18 in young women: an interim analysis of a phase III double-blind, randomised controlled trial. Lancet 369: 2161-2170.
18. Garland SM, Hernandez-Avila M, Wheeler CM, Perez G, Harper DM, Leodolter S, Tang GW, Ferris DG, Steben M, Bryan J, Taddeo FJ, Railkar R, Esser MT, Sings HL, Nelson M, Boslego J, Sattler C, Barr E, Koutsky LA, Females United to Unilaterally Reduce Endo/Ectocervical Disease II. (2007) Quadrivalent vaccine against human papillomavirus to prevent anogenital diseases. New England J Med 356: 1928-1943.
19. Villa LL, Costa RL, Petta CA, Andrade RP, Ault KA, Giuliano AR, Wheeler CM, Koutsky LA, Malm C, Lehtinen M, Skjeldestad FE, Olsson SE, Steinwall M, Brown DR, Kurman RJ, Ronnett BM, Stoler MH, Ferenczy A, Harper DM, Tamms GM, Yu J, Lupinacci L, Railkar R, Taddeo FJ, Jansen KU, Esser MT, Sings HL, Saah AJ, Barr E. (2005) Prophylactic quadrivalent human papillomavirus (types 6, 11, 16, and 18) L1 virus-like particle vaccine in young women: a randomised double-blind placebo-controlled multicentre phase II efficacy trial. Lancet Oncology 6: 271-278.
20. Schroepfer G.J.Jr. (2000) Oxysterols: modulators of cholesterol metabolism and other processes. Physiol Rev 80: 361-554.
21. Leonarduzzi G, Sottero B, Poli G. (2002) Oxidized products of cholesterol: dietary and metabolic origin, and proatherosclerotic effects. J Nutr Biochem 13: 700-710.
22 Poli G, Biasi F, Leonarduzzi G (2013) Oxysterols in the pathogenesis of major chronic diseases. Redox Biology 1: 125-130.
23 Lester SN, Li K. (2014) Toll-like receptors in antiviral innate immunity. J Mol Biol 426; 1246-1264.
24. Testa G, Gamba P, Badilli U, Gargiulo S, Maina M, Guina T, Calfapietra S, Biasi F, Cavalli R, Poli G, Leonarduzzi G. (2014) Loading into nanoparticles improves quercetin's efficacy in preventing neuroinflammation induced by oxysterols. PLoS One 9: e96795.
25. Kawai T, Akira S. (2011) Toll-like receptors and their crosstalk with other innate receptors in infection and immunity. Immunity 34: 637-650.
26. Poli G, Sottero B, Gargiulo S, Leonarduzzi G. (2009) Cholesterol oxidation products in the vascular remodeling due to atherosclerosis. Mol Aspects Med 30: 180-189.
27. Coulson BS, Fowler KJ, Bishop RF, Cotton RG. (1985) Neutralizing monoclonal antibodies to human rotavirus and indications of antigenic drift among strains from neonates. J Virol 54: 14-20.
28. Graham KL, Zeng W, Takada Y, Jackson DC, Coulson BS. (2004) Effects on rotavirus cell binding and infection of monomeric and polymeric peptides containing alpha2beta1 and alphaxbeta2 integrin ligand sequences. J Virol. 78(21): 11786-97.
29. Livak JK, Schmittgen TD (2001) Analysis of relative gene expression data using real-time quantitative PCR and the 2(-Delta Delta C(T)) method. Methods 25: 402-408.
30. Amini-Bavil-Olyaee S, Choi YJ, Lee JH, Shi M, Huang IC, Farzan M, Jung JU. The antiviral effector IFITM3 disrupts intracellular cholesterol homeostasis to block viral entry. Cell Host Microbe. 2013 Apr 17;13:452-64.
31. Poh MK, Shui G, Xie X, Shi PY, Wenk MR, Gu F. U18666A, an intra-cellular cholesterol transport inhibitor, inhibits dengue virus entry and replication. Antiviral Res. 2012 Jan;93:191-8.
32. Roulin PS, Lötzerich M, Torta F, Tanner LB, van Kuppeveld FJ, Wenk MR, Greber UF. Rhinovirus uses a phosphatidylinositol 4-phosphate/cholesterol countercurrent for the formation of replication compartments at the ER-Golgi interface. Cell Host Microbe. 2014 Nov 12;16(5):677-90.

## Claims

1. Oxysterol of formula (I): 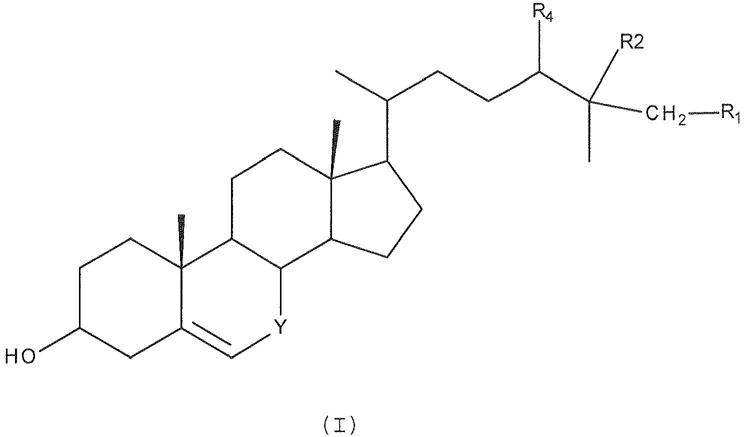 wherein R1 is chosen from the group consisting of H and OH, R2 is chosen from the group consisting of H and OH, R4 is chosen from the group consisting of H, OH, Y is chosen from the group consisting of CHR3 and C=O, and R3 is chosen from the group consisting of H and OH, for use in the treatment or in the prevention of infections caused by a naked virus selected from the group consisting of rotavirus, papillomavirus and rhinovirus.

2. Oxysterol for use in the treatment of infections caused by naked viruses according to claim 1, wherein said infection is selected from the group consisting of gastroenteritis, rhinopharyngitis, asthma exacerbation, chronic obstructive pulmonary disease exacerbation, genital condyloma, cervical cancer, vulvar cancer, vaginal cancer, penile cancer, anal cancer, perianal cancer, head cancer, neck cancer, warts, papillomas, recurrent respiratory papillomatosis.

3. Oxysterol for use in the treatment of infections caused by naked viruses according to any one of the preceding claims, wherein said oxysterol is 27-hydroxycholesterol.

4. Oxysterol for use in the treatment of infections caused by naked viruses according to any one of the claims 1 or 2, wherein said oxysterol is 7β-hydroxycholesterol.

5. Oxysterol for use in the treatment of infections caused by naked viruses according to any one of the claims 1 or 2, wherein said oxysterol is 7-ketocholesterol.

6. Oxysterol for use in the treatment of infections caused by naked viruses according to any one of the claims 1 or 2, wherein said oxysterol is 25-hydroxycholesterol.

7. Oxysterol for use in the treatment of infections caused by naked viruses according to any one of the claims 1 or 2, wherein said oxysterol is 7α-hydroxycholesterol.

8. Oxysterol for use in the treatment of infections caused by naked viruses according to any one of the claims 1 or 2, wherein said oxysterol is 24-hydroxycholesterol.

9. Oxysterol for use in the treatment of infections caused by naked viruses according to any one of the claims 1 or 2, wherein said oxysterol is 7alpha,25-di hydroxycholesterol.

10. Pharmaceutical composition comprising an oxysterol of formula (I): 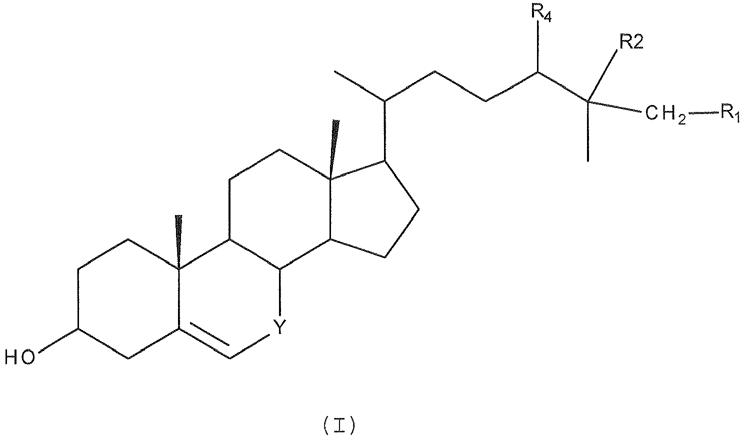 wherein R1 is chosen from the group consisting of H and OH, R2 is chosen from the group consisting of H and OH, R4 is chosen from the group consisting of H, OH, Y is chosen from the group consisting of CHR3 and C=O, and R3 is chosen from the group consisting of H and OH, and at least one pharmaceutically acceptable excipient for use in the treatment or prevention of infections caused by a naked virus selected from the group consisting of rotavirus, papillomavirus and rhinovirus.

## Patentansprüche

1. Oxysterol mit der Formel (I) : 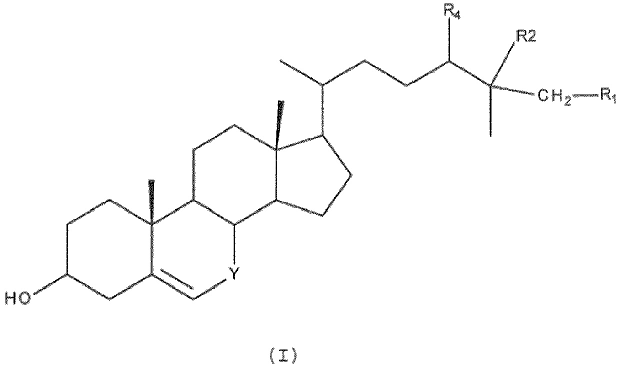 wobei R1 ausgewählt ist aus der Gruppe bestehend aus H und OH, R2 ausgewählt ist aus der Gruppe bestehend aus H und OH, R4 ausgewählt ist aus der Gruppe bestehend aus H, OH, Y ausgewählt ist aus der Gruppe bestehend aus CHR3 und C=0, und R3 ausgewählt ist aus der Gruppe bestehend aus H und OH, zur Verwendung bei der Behandlung oder Prävention von Infektionen, die durch einen nackten Virus verursacht werden, ausgewählt aus der Gruppe bestehend aus Rotavirus, Papillomavirus und Rhinovirus.

2. Oxysterol zur Verwendung bei der Behandlung von Infektionen, die durch nackte Viren nach Anspruch 1 verursacht werden, wobei die Infektion ausgewählt ist aus der Gruppe bestehend aus Gastroenteritis, Rhinopharyngitis, Asthma-Exazerbation, chronisch obstruktiver Lungenerkrankungs-Exazerbation, Genitalkondylom, Gebärmutterhalskrebs, Vulvakrebs, Vaginalkrebs, Peniskrebs, Analkrebs, Perianalkrebs, Kopfkrebs, Halskrebs, Warzen, Papillomen, rezidivierende respiratorische Papillomatose.

3. Oxysterol zur Verwendung bei der Behandlung von Infektionen, die durch nackte Viren gemäß einem der vorhergehenden Ansprüche verursacht werden, wobei das Oxysterol 27-Hydroxycholesterin ist.

4. Oxysterol zur Verwendung bei der Behandlung von Infektionen, die durch nackte Viren nach einem der Ansprüche 1 oder 2 verursacht werden, wobei das Oxysterol 7β-Hydroxycholesterin ist.

5. Oxysterol zur Verwendung bei der Behandlung von Infektionen, die durch nackte Viren nach einem der Ansprüche 1 oder 2 verursacht werden, wobei das Oxysterol 7-Ketocholesterin ist.

6. Oxysterol zur Verwendung bei der Behandlung von Infektionen, die durch nackte Viren nach einem der Ansprüche 1 oder 2 verursacht werden, wobei das Oxysterol 25-Hydroxycholesterin ist.

7. Oxysterol zur Verwendung bei der Behandlung von Infektionen, die durch nackte Viren nach einem der Ansprüche 1 oder 2 verursacht werden, wobei das Oxysterol 7α-Hydroxycholesterin ist.

8. Oxysterol zur Verwendung bei der Behandlung von Infektionen, die durch nackte Viren nach einem der Ansprüche 1 oder 2 verursacht werden, wobei das Oxysterol 24-Hydroxycholesterin ist.

9. Oxysterol zur Verwendung bei der Behandlung von Infektionen, die durch nackte Viren nach einem der Ansprüche 1 oder 2 verursacht werden, wobei das Oxysterol 7alpha,25-Dihydroxycholesterin ist.

10. Pharmazeutische Zusammensetzung, umfassend ein Oxysterol mit der Formel (I) : 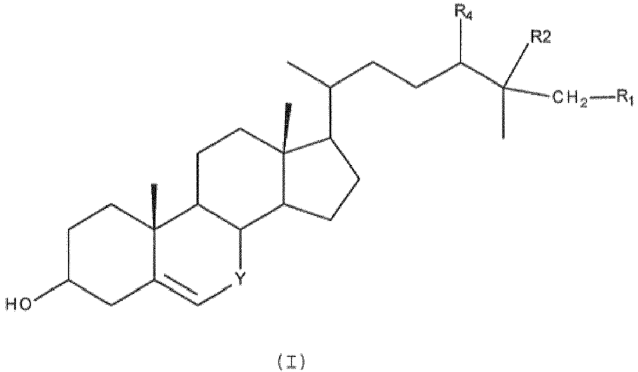 wobei R1 ausgewählt ist aus der Gruppe bestehend aus H und OH, R2 ausgewählt ist aus der Gruppe bestehend aus H und OH, R4 ausgewählt ist aus der Gruppe bestehend aus H, OH, Y ausgewählt ist aus der Gruppe bestehend aus CHR3 und C=0, und R3 ausgewählt ist aus der Gruppe bestehend aus H und OH, und mindestens einen pharmazeutisch annehmbaren Hilfsstoff zur Verwendung bei der Behandlung oder Prävention von Infektionen, die durch einen nackten Virus verursacht werden, ausgewählt aus der Gruppe bestehend aus Rotavirus, Papillomavirus und Rhinovirus.

## Revendications

1. Oxystérol de formule (I) : 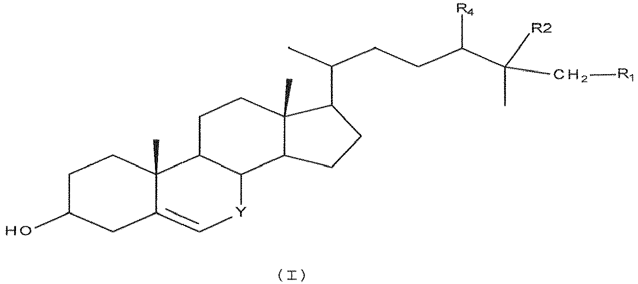 où R1 est choisi dans le groupe constitué par H et OH, R2 est choisi dans le groupe constitué par H et OH, R4 est choisi dans le groupe constitué par H, OH, Y est choisi dans le groupe constitué par CHR3 et C = O, et R3 est choisi dans le groupe constitué par H et OH, pour une utilisation dans le traitement ou la prévention d'infections causées par un virus nu choisi dans le groupe constitué par les rotavirus, les papillomavirus et les rhinovirus.

2. Oxystérol pour une utilisation dans le traitement d'infections causées par des virus nus selon la revendication 1, dans lequel ladite infection est choisie dans le groupe constitué par les gastro-entérite, rhinopharyngite, exacerbation de l'asthme, exacerbation de la maladie pulmonaire obstructive chronique, condylomes génitaux, cancer du col utérin, cancer de la vulve, cancer vaginal, cancer du pénis, cancer anal, cancer périanal, cancer de la tête, cancer du cou, verrues, papillomes, papillomatoses respiratoires récurrentes.

3. Oxystérol pour une utilisation dans le traitement d'infections causées par des virus nus selon l'une quelconque des revendications précédentes, dans lequel ledit oxystérol est le 27-hydroxycholestérol.

4. Oxystérol pour une utilisation dans le traitement d'infections causées par des virus nus selon l'une quelconque des revendications 1 ou 2, dans lequel ledit oxystérol est le 7β-hydroxycholestérol.

5. Oxystérol pour une utilisation dans le traitement d'infections causées par des virus nus selon l'une quelconque des revendications 1 ou 2, dans lequel ledit oxystérol est le cétocholestérol.

6. Oxystérol pour une utilisation dans le traitement d'infections causées par des virus nus selon l'une quelconque des revendications 1 ou 2, dans lequel ledit oxystérol est le 25-hydroxycholestérol.

7. Oxystérol pour une utilisation dans le traitement d'infections causées par des virus nus selon l'une quelconque des revendications 1 ou 2, dans lequel ledit oxystérol est le 7α-hydroxycholestérol.

8. Oxystérol pour une utilisation dans le traitement d'infections causées par des virus nus selon l'une quelconque des revendications 1 ou 2, dans lequel ledit oxystérol est le 24-hydroxycholestérol.

9. Oxystérol pour une utilisation dans le traitement d'infections causées par des virus nus selon l'une quelconque des revendications 1 ou 2, dans lequel ledit oxystérol est le 7alpha, 25-di hydroxycholestérol.

10. Composition pharmaceutique comprenant un oxystérol de formule (I) : 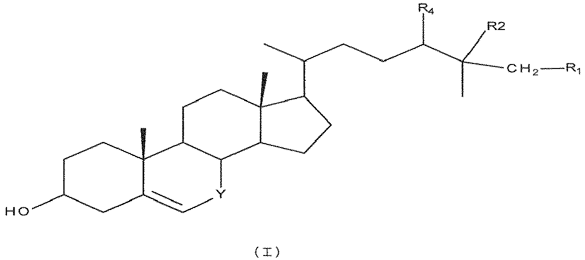 dans laquelle R1 est choisi dans le groupe constitué par H et OH, R2 est choisi dans le groupe constitué par H et OH, R4 est choisi dans le groupe constitué par H, OH, Y est choisi dans le groupe constitué par CHR3 et C = O, et R3 est choisi dans le groupe constitué par H et OH, et au moins un excipient pharmaceutiquement acceptable pour une utilisation dans le traitement ou la prévention d'infections causées par un virus nu choisi dans le groupe comprenant les rotavirus, les papillomavirus et les rhinovirus.
